# EUROPEAN PATENT SPECIFICATION

(11) **EP 3 359 213 B1**
(45) Date of publication and mention of the grant of the patent: **23.02.2022**
(21) Application number: 16854309.8
(22) Date of filing: 06.10.2016
(51) Int. Cl.: A61L 27/52, C12N 5/071, C12N 5/02

(54) **HIGH-THROUGHPUT PLATFORM FOR BIOPRINTING TISSUE MODULES**
PLATTFORM MIT HOHEM DURCHSATZ FÜR BIOPRINTING VON GEWEBEMODULEN
PLATE-FORME À HAUT RENDEMENT PERMETTANT LA BIO-IMPRESSION DE MODULES TISSULAIRES

(30) Priority: 06.10.2015 US 201562237843 P
(43) Date of publication of application: 15.08.2018
(73) Proprietor: University Of South Florida, Tampa, FL 33512-9220 (US)
(72) Inventor: CROSS, Michael C., Tampa, FL 33620-3068 (US); AKINTEWE, Olukemi O., Tampa, FL 33607-4174 (US); DUPONT, Samuel James, Tampa, FL 33618 (US); ELINENI, Kranthi Kumar, Hillsboro, OR 97124 (US); TOOMEY, Ryan G., Tampa, FL 33647 (US); GALLANT, Nathan D., Tampa, FL 33629 (US)
(74) Representative: HGF
(86) International application number: PCT/US2016/055718
(87) International publication number: WO 2017/062600

(56) References cited:
- Olukemi O Akintewe ET AL: "Fabrication of Tissue Precursors Induced by Shape-changing Hydrogels", , 1 January 2015 (2015-01-01), XP055550461, ISBN: 978-1-321-92879-2 Retrieved from the Internet: URL:https://scholarcommons.usf.edu/cgi/vie wcontent.cgi?article=6829&context=etd
- KIKUCHI TETSUTARO ET AL: "Automatic fabrication of 3-dimensional tissues using cell sheet manipulator technique", BIOMATERIALS, ELSEVIER SCIENCE PUBLISHERS BV., BARKING, GB, vol. 35, no. 8, 23 December 2013 (2013-12-23), pages 2428-2435, XP028814366, ISSN: 0142-9612, DOI: 10.1016/J.BIOMATERIALS.2013.12.014
- OLUKEMI O. AKINTEWE ET AL: "Shape-changing hydrogel surfaces trigger rapid release of patterned tissue modules", ACTA BIOMATERIALIA, vol. 11, 1 January 2015 (2015-01-01), pages 96-103, XP055550102, AMSTERDAM, NL ISSN: 1742-7061, DOI: 10.1016/j.actbio.2014.09.040
- Dupont ET AL: "Shape-Shifting Surfaces for Rapid Release and Direct Stamping of Organized Micro-Tissues", , 1 January 2012 (2012-01-01), XP055550143, ISBN: 978-1-267-80214-9 Retrieved from the Internet: URL:https://scholarcommons.usf.edu/cgi/vie wcontent.cgi?article=5506&context=etd
- OLUKEMI O. AKINTEWE ET AL: "Microcontact printing of tissue precursors via geometrically patterned shape-changing hydrogel stamps preserves cell viability and organization", BIOPRINTING, vol. 8, 1 December 2017 (2017-12-01), pages 22-29, XP055550115, ISSN: 2405-8866, DOI: 10.1016/j.bprint.2017.06.001

## Description

### FIELD OF THE INVENTION

The invention provides methods for producing three-dimensional tissue structures.

### BACKGROUND OF INVENTION

Engineering robust biomimetic 3D tissue structures is crucial to facilitate the repair of diseased tissues and organs. An ideal tissue construct should be able to recapitulate the functions of complex tissue micro architecture found in natural tissues. Construction of 3D tissues *in vitro* also creates an effective design model to systematically study a variety of cell types by manipulating cell interactions through tissue assembly, patterning, or geometry.

Functioning of tissues is well correlated with the anatomic structure. Mechanical functioning of skeletal muscles is sustained by their vasculature, which is anisotropic in nature when fusion occurs between muscle fibers and myoblasts. In the organs, the structural network of liver facilitates transportation of waste and products. Each of the lobules in the liver is made up of plate-like assemblies of hepatic cells. Similarly, the highly oriented cytoarchitecture found in myocardial tissues is important for the proper electromechanical functioning of the cardiomyocytes (CM).

Existing cell bioprinting technology enables the placement of viable cells into three dimensional constructs. Living organs and tissues are formed by injection molding with patterns generated by computer-aided design and computer-aided manufacturing (CAD/CAM). These methods require intermediate structures such as hydrogels or liquid suspension. For extrusion bioprinting, cell suspensions have low viscosity and expose cells to high-shear stress when expelled through a nozzle. Cell morphology and shape-dependent functions are not retained immediately following printing and require a re-adjustment period. Patterning large areas, *e.g.* 1 mm x 1 mm, using ink-jet extrusion methods would take minimum 3 hours.

Tetsutaro Kikuchi et al. described in "Automatic fabrication of 3-dimensional tissues using cell sheet manipulator technique" (Biomaterials, 2014) an automatic cell sheet stacking apparatus to fabricate 3-dimensional tissue engineered constructs exploiting their cell sheet manipulator technique, where cell sheets harvested from temperature-responsive culture dishes are stacked into a multilayered cell sheet.

Olukemi O. Akintewe et al. described in "Shape-changing hydrogel surfaces trigger rapid release of patterned tissue modules" (Acta Biomaterialia, 2015) micro-scale surfaceattached hydrogels made of a thermo responsive polymer used as a cell culture support to fabricate tissue modules of defined geometric shape.

In the dissertation by Samuel J. DuPont, Jr. "Shape-shifting surfaces for repaid release and direct stamping of organized micro tissues" (2012) it was the aim to develop a robust and simple platform for the *in vitro* organization of cells on surfaces which facilitate rapid cell release and which allows for the direct stamping of highly organized micro-tissues.

The most prevalent challenge encountered in the current techniques is the need to enhance prevascularization in the engineered tissues. The common strategy is to engineer dual cell types in different orientations to induce structural organization and promote formation of vascular networks upon tissue implantation.

### BRIEF SUMMARY

The invention provides methods for rapid production of organized tissue precursors with different sizes and geometry.

An embodiment of the invention provides a method of producing 3D tissue structures, the method comprising producing a plurality of patterned monolayers of cells and sequentially overlaying the plurality of patterned monolayers of cells to produce a stack of monolayers of cells. The patterns of cells in the plurality of monolayers can be designed in a manner which produces a tissue or a portion of a tissue when the plurality of monolayers is overlaid into a stack. Also, different layers in the plurality of monolayers of cells can comprise two or more types of cells and the plurality of monolayers of cells can be designed in a manner which produces a tissue containing the types of cells in a naturally occurring tissue or a portion of a tissue when the plurality of monolayers is overlaid into a stack.

Also described is an apparatus for producing a 3D tissue module. The apparatus comprises: a three-dimensional positioning system, a contact stamp, a contact stamp storage container, a contact stamp holder, a contact stamp exchange unit, and a contact stamp actuator.

### BRIEF DESCRIPTION OF DRAWINGS

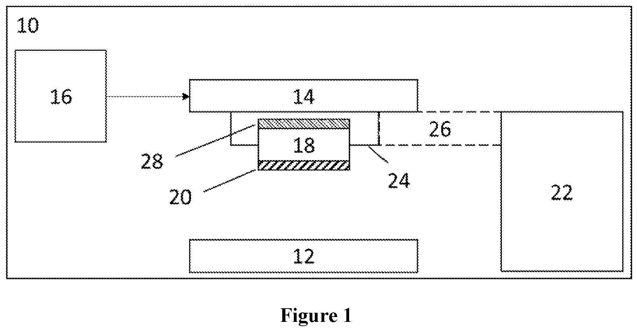

Figure 1. Schematic view of an embodiment of high-throughput platform for bioprinting tissue modules.

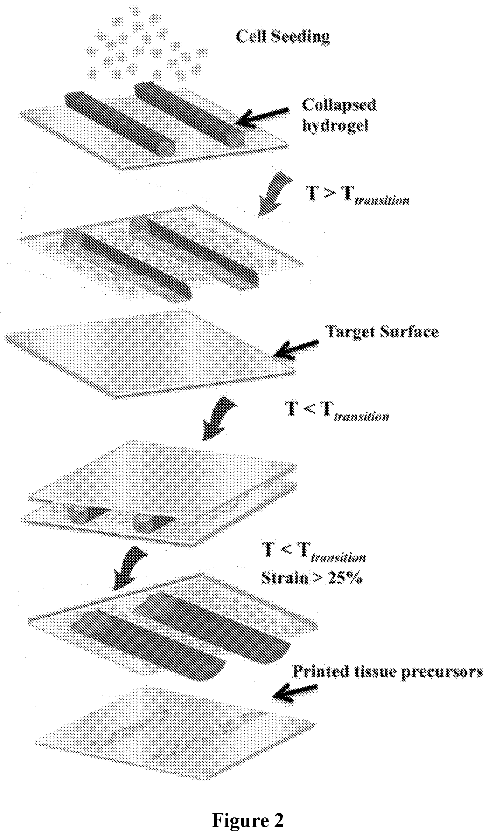

Figure 2. Schematic illustration of the formation and release of tissue precursors (from shape-changing hydrogel micro arrays). Cells adhered to and conformed to the shape of the arrays, organizing into geometric tissue modules. Tissue precursors are then released from micro arrays upon expansion beyond a critical lateral strain of 25%.

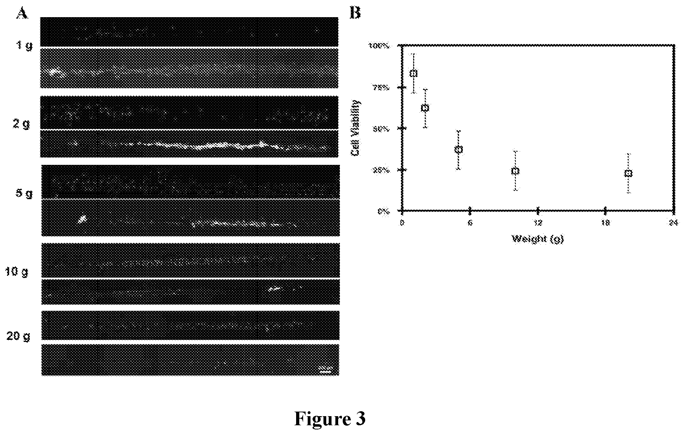

Figure 3. Viability of fibroblasts transferred from shape-changing hydrogel (different applied pressure ranging from 1 to 20 g weights). A: Fluorescence micrographs of fibroblast viability. Dead cells (red) and Live cells (green stain). B: Effects of applied pressured on cell viability. Bars represents standard error (SE) (n >3). Scale bar = 200 µm.

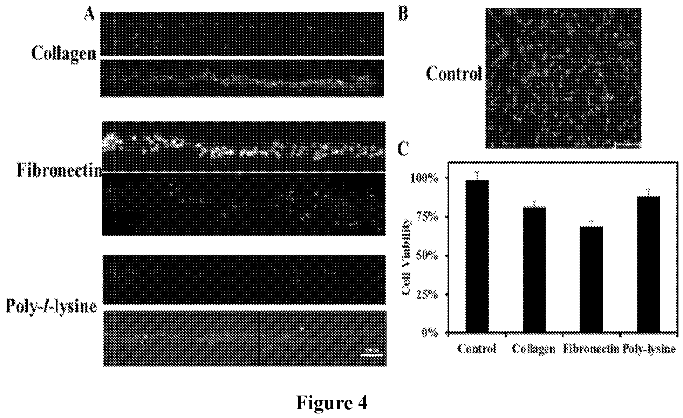

Figure 4. Tissue precursors were printed onto a variety of adhesive surfaces. (A) Fluorescence micrographs of live (green, upper) and dead (red, lower) cell images of printed tissue precursors on collagen, fibronectin and poly-L-lysine surfaces (bar = 100 µm). (B) Merged image of live (green) and dead (red) cells which were seeded on plain glass coverslips as a positive control for cell viability (bar = 50 µm). (C) Cell viability after printing onto each coating or for the control equaled the count of green cells divided by the sum of green and red cells. Data represent the mean ± 95% confidence interval for n ≥ 3 individual measurements. A ^{∗} indicates a significant difference compared to the control (p < 0.01).

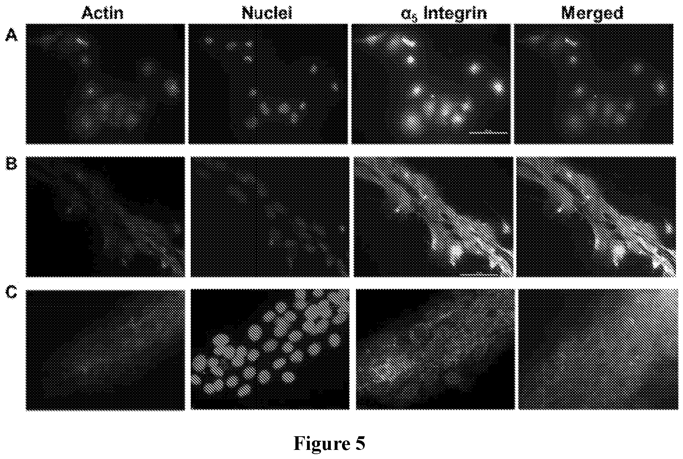

Figure 5. Structural components of fibroblast before and after printing with shape-changing hydrogel. Fluorescence micrographs of actin filament, nuclei and α5 integrin. A: After 2 hours of cell seeding on TCPS. B: Images of tissue precursors after 1 hour post printing. C: Printed fibroblasts showing formation of actin filaments, focal adhesion complexes and nuclei after 2 hours post printing. Cell nuclei (blue) were stained using Hoechst, actin filaments were stained with AF 568 phalloidin (red) and α5 integrin were stained using polyclonal ab 1928 (green). Scale bar = 50 µm.

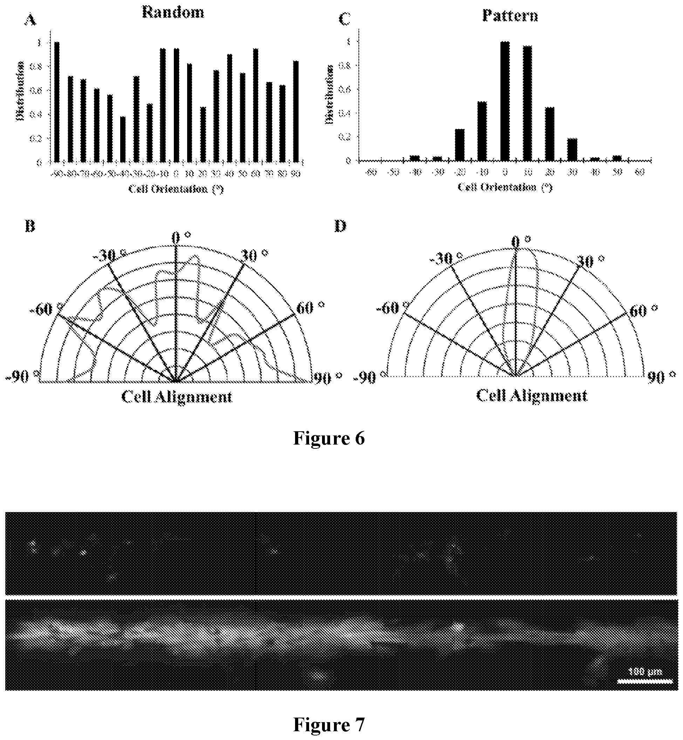

Figure 6. Cellular spatial organization before and after printing from shape-changing hydrogel. A & B: Distribution of cell orientation and polar plot on the control samples showing random organization. Cells were seeded on plain TCPS with no patterning for 24 hours. C & D: Distribution of cell orientation and polar plot on printed tissue precursors samples showing aligned organization governed by the hydrogel's pattern. Cells were seeded on patterned arrays for 24 hours with pattern direction at 0° axis. Sample size, n ≥ 3.
Figure 7. Murine skeletal C2C12 tissue precursors printed with shape-changing hydrogel. Fluorescence images of printed Myoblast tissue precursors on polylysine surfaces under applied pressure of 1g. Dead cells (red) and Live cells (green stain). Scale bar = 100 µm.

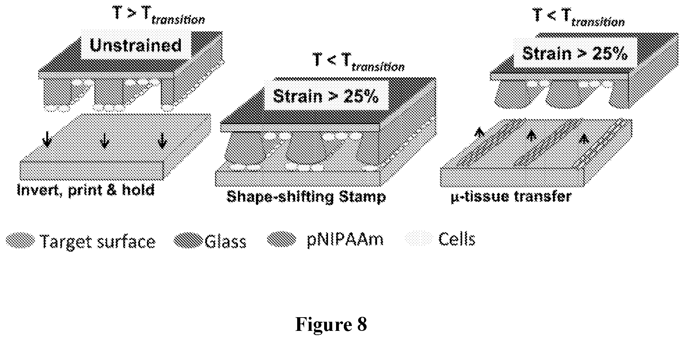

Figure 8. Schematic diagram of micro-contact printing technique. Cells are cultured on a shape-changing stamp; the stamp is inverted and placed in contact with a target surface to facilitate cell adhesion; a thermal shift induces swelling which causes rapid detachment of the tissue modules from the stamp, transferring the pattern cells to the target surface.

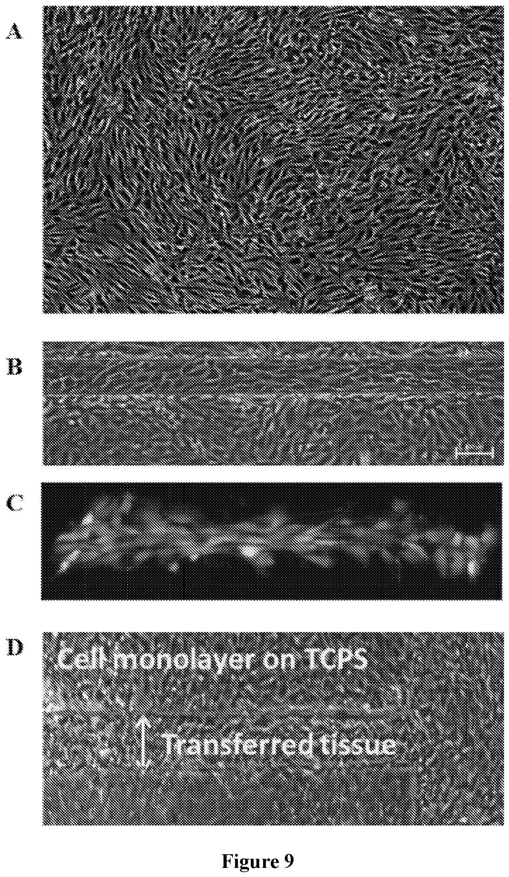

Figure 9. Fabrication of fibroblast tissue modules. (A) Confluent monolayers of cells cultured on TCPS after 24 hours. (B) Cells cultured on 100 µm wide arrays of shape-changing hydrogel. (C) Tissue modules printed on polylysine coated TCPS stained with cell tracker green. (D) Printed tissue modules on monolayer of cells.

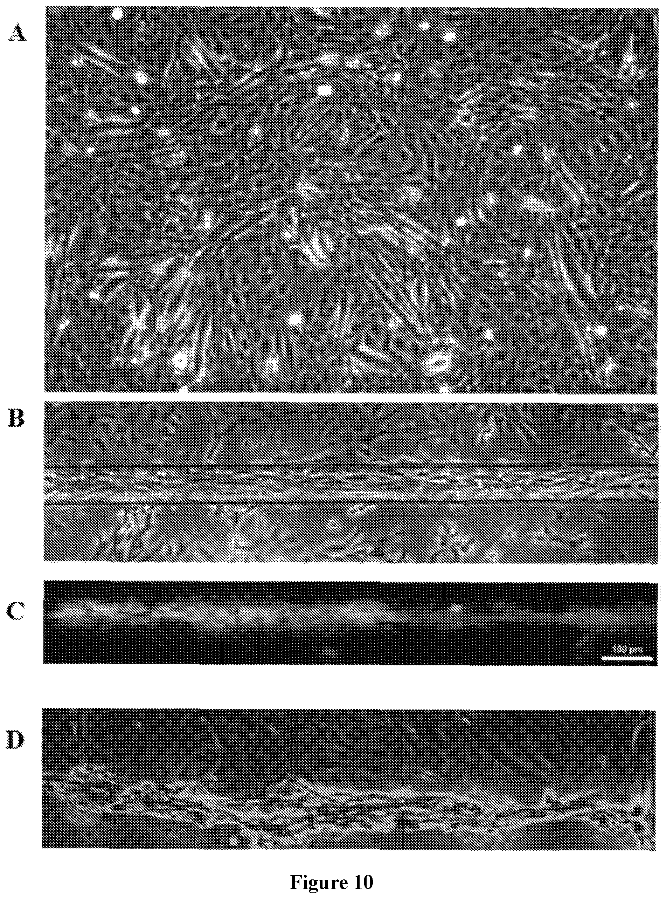

Figure 10. Fabrication of myoblast tissue modules. (A) Confluent monolayers of cells cultured on TCPS after 48 hours. (B) Cells cultured on 100 µm wide arrays of shape-changing hydrogel. (C) Tissue modules printed on polylysine coated TCPS stained with cell tracker green. (D) Printed tissue modules on monolayer of cells.

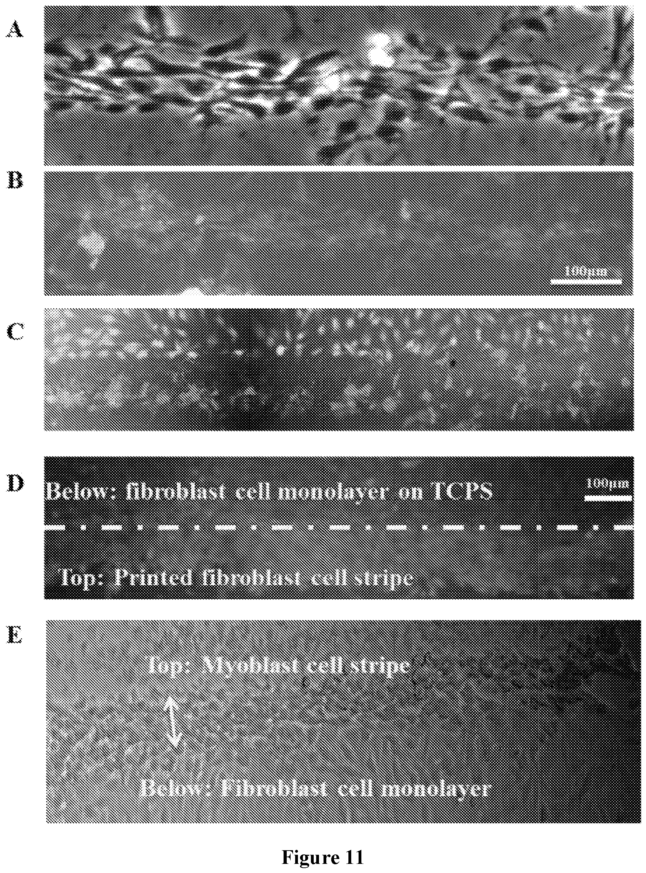

Figure 11. Assembly of 2-layer tissue modules. (A) Printed fibroblast tissue modules on monolayer of fibroblast cells in phase contrast with conformal printing pressure of 2 grams. (B) Printed fibroblast tissue modules (red stain) on monolayer of fibroblast cells (green stain), fluorescent images. (C) Printed fibroblast tissue modules on monolayer of fibroblast cells with cell tracker red staining on both layers with conformal printing pressure of 1 gram. (D) Printed myoblast tissue modules on monolayer of fibroblast cells in phase contrast.

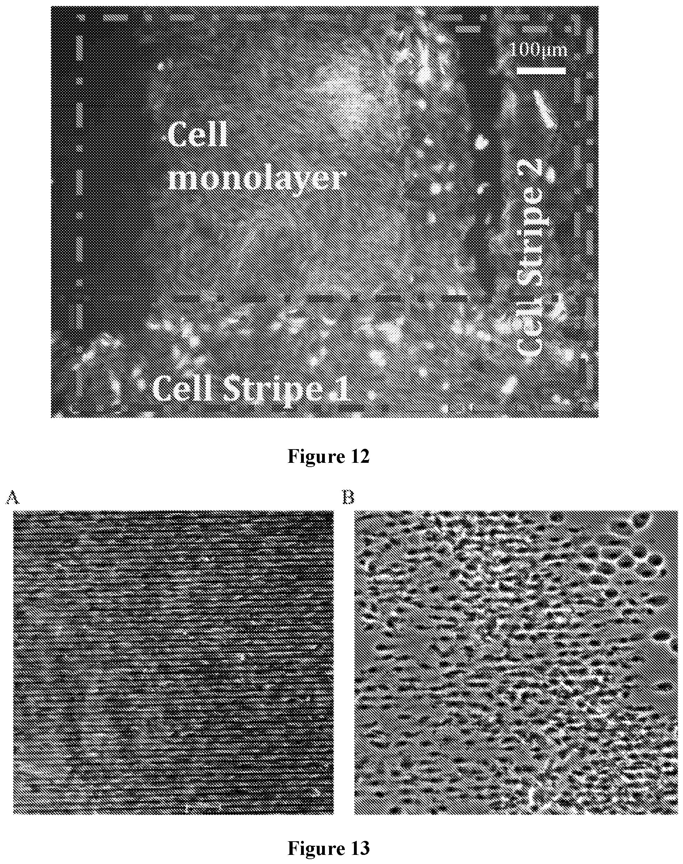

Figure 12. Assembly of 3-layer tissue modules. Monolayer of fibroblast cells (unstained) are cultured for 48 hours then myoblast cells (stained green) are printed along the horizontal axis of the monolayer as cell stripe 1, while an array of fibroblast cells (stained red) are printed on top of the cell stripe 1 along the vertical axis of the monolayer as cell stripe 2.
Figure 13. Fabrication of cells from closely spaced arrays of the shape-changing hydrogel. (A) Fibroblasts are cultured on 10 µm wide by 10 µm spacing arrays of the microbeams for 24 hours. (B) Printed cells from the 10 µm by 10 µm arrays onto a polylysine coated TCPS.

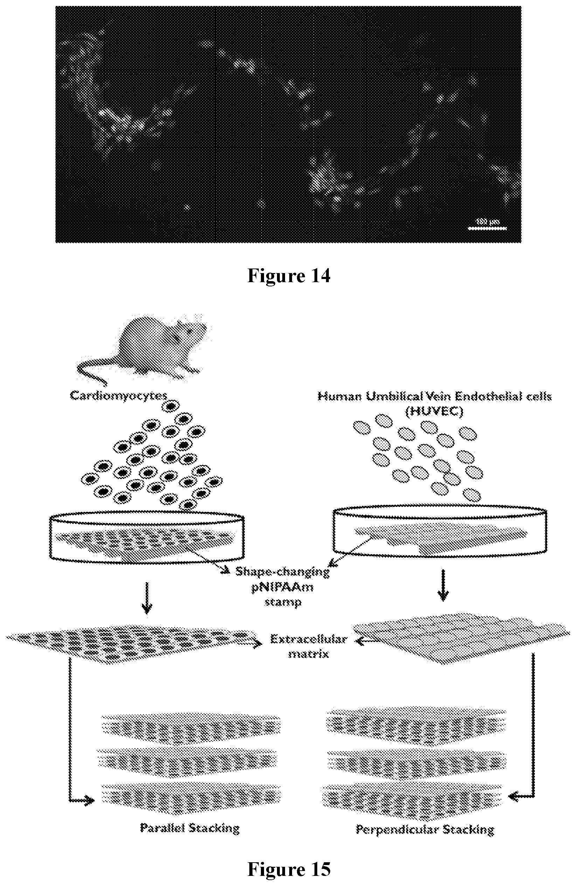

Figure 14. Fabrication of buckled tissue modules from 75 µm wide arrays of the shape-changing hydrogel. (A) Printed tissue modules on polylysine coated TCPS, calcein staining (B) Live (green) /Dead (red) staining of the buckled tissue module.
Figure 15. Neonatal rat cardiomyocytes can be cultured on shape-changing poly-(*N-*isopropylacrylamide) constructs concurrently with endothelial cells for formation of intact aligned micro-tissues. Layers of tissues can then be stacked into parallel or perpendicular orientations to promote diffusion of nutrients.

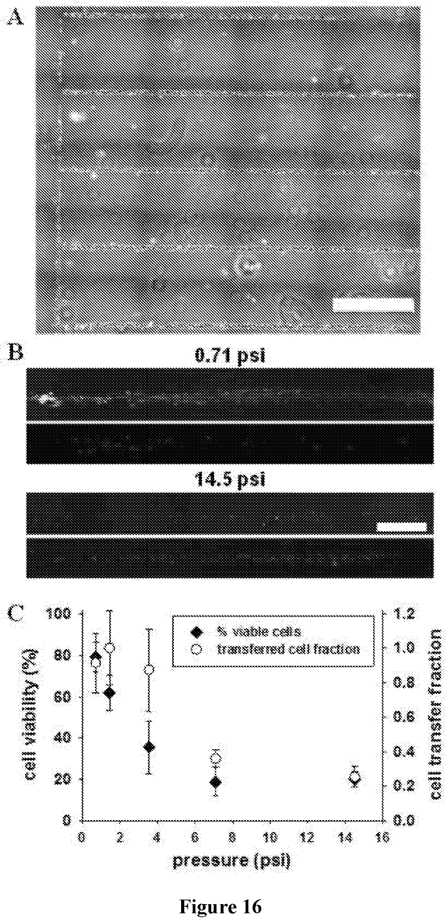

Figure 16. Tissue pattern transfer and viability depends on the pressure applied to the stamp during printing. (A) Microcontact printing from shape changing stamps directly patterns the tissue precursor geometry and cell shapes (bar = 500 *µ*m). (B) Fluorescence micrographs indicate live (green) and dead (red) cells in stripe patterns after printing under various pressures (bar = 1000 *µ*m). (C) Cell viability and transfer efficiency strongly depended on the stamping pressure used to ensure conformal contact between the stamp and the target. Viability equaled the count of green cells divided by the sum of green and red cells and the transfer fraction was the sum of green and red cells normalized to the average initial count. Data represent the mean ± 95% confidence interval for n ≥ 7 individual measurements.

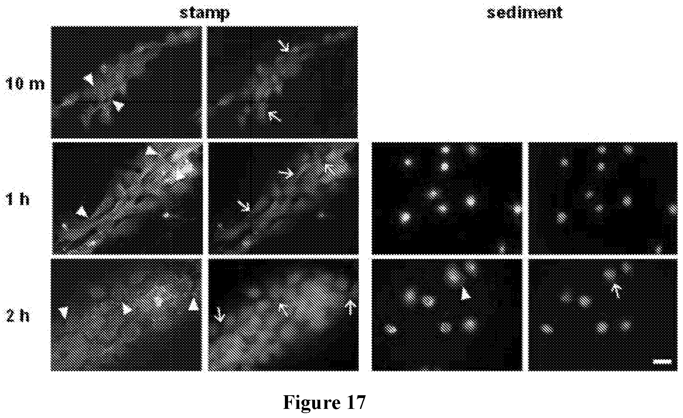

Figure 17. Cytoskeletal maintenance and rapid adhesion complex assembly after printing were distinct from the reorganization that occurs in sedimentation seeded cells. Fluorescence micrographs of actin filaments (red), nuclei (blue) and α5 integrins (green) for printed and control samples after 10 min, 1 h and 2 h showed unique adhesion and organization evolutions. Solid arrow heads indicate clustering integrins in adhesion complexes and arrows specify actin stress fibers (bar = 25 µm).

### DETAILED DISCLOSURE

In the following detailed description, reference is made to the accompanying drawings, depicting exemplary, non-limiting and non-exhaustive embodiments of the invention. These embodiments are described in sufficient detail to enable those having skill in the art to practice the invention, and it is understood that other embodiments may be used, and other changes may be made, without departing from the spirit or scope of the invention. The following detailed description is, therefore, not to be taken in a limiting sense, and the scope of the invention is defined only by the appended claims. Like numbers refer to similar features of like elements throughout.

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising". The transitional terms/phrases (and any grammatical variations thereof) "comprising", "comprises", "comprise", "consisting essentially of', "consists essentially of', "consisting" and "consists" can be used interchangeably.

The term "about" or "approximately" means within an acceptable error range for the particular value as determined by one of ordinary skill in the art, which will depend in part on how the value is measured or determined, *i.e.,* the limitations of the measurement system. For example, "about" can mean within 1 or more than 1 standard deviation, per the practice in the art. Alternatively, "about" can mean a range of up to 0-20%, 0 to 10%, 0 to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed. In the context of compositions containing amounts of ingredients where the terms "about" or "approximately" are used, these compositions contain the stated amount of the ingredient with a variation (error range) of 0-10% around the value (X±10%).

In one embodiment, the invention provides a method of producing tissue modules, the method comprising:
a) seeding a basal surface with a first group of cells and incubating the first group of cells on the basal surface for a sufficient period of time to allow the first group of cells to adhere to the basal surface, for example, approximately 24 hours, and form a first layer of cells;
b) seeding a thermo-responsive hydrogel stamp with a second group of cells and incubating the second group of cells on the stamp for sufficient period of time to allow the second group of cells to adhere to the stamp and form a second layer of cells;
c) contacting the stamp to the basal surface, for example, with a weight applied on to the stamp or pressing the stamp and the basal surface together;
d) releasing the cells from the stamp by applying a change in temperature;
e) incubating the stamp and the basal surface for sufficient period of time, for example, for about 2 to 15 minutes, for the second layer of cells from the stamp to adhere to the first layer of cells on the basal surface;
f) separating the stamp and the basal surface to release the tissue module wherein the method further comprises repeating b) to f) with a plurality of layers of cells and stacking the plurality of layers of cells onto layers of cells previously produced.

For the purpose of this invention, the term "tissue module" refers to an arrangement of cells, for example, the arrangement of at least two layers of cells, which mimic the arrangement of cells similar to or identical to a tissue or an organ naturally found in an animal. For example, a tissue module can comprise two layers of cells that mimic two layers of cells in a skin of an animal. A tissue module can also comprise a plurality of layers of cells arranged in a manner that mimics an organ from an animal.

The cells can be incubated after steps a) and/or b) to allow the cells to multiple.

This procedure provides a structure containing two layers of cells, and is repeated to produce a structure containing a plurality of cells stacked upon each other. In one embodiment, the plurality of cells stacked upon each other mimic the arrangement of cells in an organ.

In a further embodiment, the invention discloses methods for fabricating tissue modules at a high throughput rate. A higher throughput rate can be accomplished through use of a plurality of stamps having pre-patterned layers of cells with the desired tissue architectures, for example, an arrangement which mimics the arrangement of cells in a tissue or an organ.

In one embodiment, different layers of cells comprise different types of cells, for example, in an arrangement mimicking the layers of different types of cells in a natural tissue. Non-limiting examples of tissues or organs which can be produced by the methods of the invention include, pineal gland, pituitary gland, thyroid gland, parathyroid gland, heart, lung, esophagus, thymus, adrenal gland, gall bladder, urinary bladder, large intestine, small intestine, kidney, liver, pancreas, spleen, stroma, prostate, testes, ovaries, uterus, muscle, and adipose.

In a further embodiment, an organ or a tissue to be produced is schematically dissected in a plurality of layers, for examle, in a computer model. A plurality of layers containing different types of cells can then be produced using various hydrogel stamps providing the arrangement of cells which corresponds to various layers of cells as depicted in the dissected plurality of layers. The plurality of layers can then be stacked together to produce a tissue structure similar to the organ or tissue upon which the plurality of layers were modeled.

In another embodiment, the design of the stimulus responsive hydrogen stamp produces a pattern that mimics the arrangement of a layer of cells in a natural tissue. For example, a layer of cells in liver comprises various types of cells arranged in different geometries.

In a further embodiment, the stimulus responsive hydrogel comprises a polymer responsive to changes in temperature, pH, ionic strength, solvent, salt, surfactant, or an electric or magnetic field.

In one embodiment, the basal surface comprises poly(dimethyl siloxane) (PDMS). In another embodiment, the basal surface is coated with an extracellular matrix component before seeding with cells, for example, fibronectin, laminin, collagen, elastin, gelatin, heparan sulfate, chondroitin sulfate, keratan sulfate, hyaluronic acid. Additional examples of extracellular matrix proteins suitable for coating the basal surface are well known to a person of ordinary skill in the art and such embodiments are within the purview of the invention.

In some embodiments, the stimulus-responsive hydrogel stamp is modified to have a relief pattern. The release of cells from the hydrogel stamp, in some embodiments, is accomplished by the application of a stimulus.

Also disclosed is an apparatus for producing 3D tissue modules. The apparatus comprises: a three-dimensional positioning system, a contact stamp, a contact stamp storage container, a contact stamp holder, a contact stamp exchange unit, and a contact stamp actuator.

The three-dimensional positioning system can comprise a support stage, a three-dimensional positioning unit, and a controller. In some embodiments, the contact stamp comprises a stimuli-responsive hydrogel modified with a relief pattern. In an embodiment, the contact stamp storage container comprises an incubator system capable of controlling environmental parameters. In some embodiments, the contact stamp holder comprises a device that performs a release/secure mechanism on the contact stamp and is connected to the three-dimensional positioning system. In some embodiments, the contact stamp exchange unit mechanically transfers the contact stamp between the storage container and the contact stamp holder. The contact stamp actuator, in some embodiments, comprises a stimulus source tailored to the type of stimulus-responsive hydrogel employed for the contact stamp. The actuator is operatively associated with the contact stamp and integrated into the contact stamp holder.

An example of an apparatus according to the disclosure is depicted in Figure 1. The apparatus depicted in Figure 1 comprises: a three-dimensional positioning system **16,** a contact stamp **18,** a contact stamp storage container **22,** a contact stamp holder **24,** a contact stamp exchange unit **26,** and a contact stamp actuator **28.**

The three-dimensional positioning system **16** further comprises: a support stage, a three-dimensional positioning unit, and a controller. Methods of using a three-dimensional positioning system to print viable cells are known in the relevant field and described in, for example, U.S. Patent Application Publication Nos. 2012-0089238 and 2009-0208466.

The contact stamp **18** comprises a stimulus-responsive hydrogel modified with a relief pattern of protruding features **20.** Hydrogels comprising stimulus-responsive polymers have been used as platforms for generating tissue modules capable of being manipulated for cellculture interactions on culture substrates. In an exemplary embodiment, the hydrogel substrate may comprise thermally responsive hydrogel materials, such as pNIPAAm. The presence of a relief pattern, in some embodiments, can improve adhesion of cells onto the hydrogel stamp.

The contact stamp storage container **22,** in some embodiments, comprises an incubator system capable of controlling environmental parameters. Examples of environmental parameters necessary to the vitality of cells include temperature and carbon dioxide. In some embodiments, the contact stamp holder **24,** connected to the three-dimensional positioning system **16,** comprises a device that secures or releases the contact stamp **18.** In some embodiments, the contact stamp exchange unit **26** mechanically transfers the contact stamp between the storage container **22** and the contact stamp holder **24.** The contact stamp actuator **28,** in some embodiments, comprises a stimulus source tailored to the type of stimulus-responsive hydrogel employed for the contact stamp. The actuator is operatively associated with the contact stamp **18** and integrated into the contact stamp holder **24.**

Additional non-limiting examples of three dimensions positioning system are provided in US Patent Nos. 5,149,548, 5,059,266, and 5,633,021,

### Materials and Methods

### Materials

Mouse embryonic fibroblasts (NIH3T3) and mouse skeletal myoblasts (C2C12) were purchased from American Type Culture Collection. Dulbecco's Modified Eagles Medium (DMEM), Dulbecco's phosphate buffered saline (DPBS), newborn calf serum (NCS), 0.25% trypsin EDTA (IX), penicillin, streptomycin, Calcein AM, ethidium homodimer, Alexa Fluor^{®} 546 phalloidin, Hoechst 33342, goat anti-rabbit IgG secondary antibody and human plasma fibronectin were purchased from Life Technologies. N-isopropylacrylamide (NIPAAm), 2-dimethoxy-2-phenylacetophenone (DMPA), N,N'-methylenebisacrylamide (MBAm), 3-(trichlorosilyl) propyl methacrylate (TPM), acetone, carbon tetrachloride, Triton X-100, formaldehyde, sodium azide (NaN3) and poly-L-lysine (PLL) were all purchased from Sigma-Aldrich. Tissue culture polystyrene (TCPS) dishes were obtained from Fischer Scientific. Methacryloxyethyl thiocarbonyl rhodamine B (polyfluor^{®} 570) was purchased from Polysciences. Silicone elastomer (PDMS) kits (Sylgard^{®}184) were obtained from Dow Corning. Bovine collagen type 1 was obtained from Advanced BioMatrix. Anti-alpha 5 integrin antibody (AB1928) was purchased from Merck Millipore.

### Micro-contact Printing Process

Initiation of tissue printing occurs once cells seeded on the arrays have reach confluence, either 24 or 48 hours. The arrays are inverted like ink stamps and are placed on top of a target surface to transfer the attached tissues (Figure 2). Prior to printing, the target surface, a glass coverslip, is coated with either of these three cell adhesion molecules (CAM): 10 µg/mL of human plasma fibronectin for 30 minutes, 100 µg/mL of bovine collagen type I for 3 hours, or 0.1 mg/mL of PLL for 5 minutes all at room temperature. The patterned hydrogel arrays used as cell culture supports resembled a stamp with a relief pattern and a tissue precursor ink. The stamp was inverted so that the attached cells faced the target and then placed on top of the target surface within a 35mm TCPS dish to transfer the attached tissues (Figure 2). To enable conformal contact to the target surface, a compressive load is applied to the back of the stamp. The sample is then incubated at 37°C for 15 minutes and immediately placed in a 4°C environment for 5 minutes to initiate the release and printing of the tissues. Finally, the stamp is gently detached from the target surface leaving behind the tissues on the target substrate.

### Stamp Force Analysis

Compressive force analysis was determined as the amount of pressure applied to the hydrogel arrays (stamp) necessary to induce printing onto the target surface. To establish the optimum load required, calibration weights in 20g, 10g, 5g, 2g, and 1g were independently applied to the backside of the stamp while in contact with the target to mate the two surfaces. The corresponding compressive pressure of each calibration weight on a 50 µm microbeam width stamp is 0.71, 1.45, 3.55, 7.10, 14.5 psi, respectively. After 5 minutes at 4°C, the load and stamp were removed and viability of the printed cells was examined.

Normal pressure was applied to the stamp to ensure conformal contact with the target by adding weight to the stamp. Compressive pressure was determined as the amount of force applied to the stamp divided by the protruding area of the hydrogel arrays in the collapsed state. Mass balance calibration weights were independently applied to the backside of the stamp while it was in contact with the target to mate the two surfaces. These weights induced a compressive pressure on the transferring cells ranging from 0.71 psi to 14.5 psi. After 5 minutes at 4°C, the load and stamp were gently removed and the viability, morphology, and transfer efficiency of the printed cells were examined. As a control, stamps that did not change lateral dimensions when cooled to 4°C were also used under the same conditions.

### Cell Viability Assay

Viability of printed cells was determined by using a LIVE/DEAD kit following the commercially recommended protocol. Briefly, cells were incubated in 200 µL of 20 µM Calcein AM and 40 µM ethidium homodimer-1 solution. Then the cells were rinsed twice with DPBS after 30 minutes and re-incubated in fresh serum containing medium prior to imaging.

### Immunofluorescence Staining for Focal Adhesion Components

Alpha 5 integrin-containing focal adhesions and actin filaments were visualized by immunofluorescence. Printed tissue precursors were fixed in ice cold 3.7% formaldehyde for 5 minutes followed by permeabilization and stabilization of cytoskeletons (CSK) in ice cold CSK buffer with Triton X-100 for 10 minutes. Then a blocking buffer composed of 5% NCS and 0.01% NaN₃ in complete DPBS was used to block nonspecific adsorption for at least 1 hour. The sample was labeled with a primary antibody, polyclonal rabbit antiintegrin alpha 5 antibody diluted in blocking buffer at 1:100 for 1 hour, and washed thrice with DPBS for 5 minutes each. It was then incubated in goat anti-rabbit IgG secondary antibody conjugated with Alexa Fluor ^{®} 488, Hoechst, phalloidin Alexa Fluor ^{®} 568 diluted in blocking buffer for 1 hour at 1:100, 1:2000, and 1:50 dilutions, respectively. Samples were rinsed three times with DPBS and twice with deionized water, sealed with mounting media, and then imaged.

### Cell Alignment and Orientation Analyses

Cell orientation of the printed tissue precursors and control samples were performed using ImageJ v1.49s (NIH, USA) with a sample size of n ≥ 3. The angle between the cell morphology and the longitudinal direction of the micro array was measured. For the control samples (cells on non-patterned arrays), this angle was between the minor and major axis of the cells. The percentage of aligned cells was analyzed from the measured orientation distribution by using Excel Analysis toolpak. Cell alignment was determined as the average amount of cells oriented within ± 20° of the preferred angle of orientation *i.e.* longitudinal axis of the hydrogel array.

### Fluorescence Imaging and Analysis

Prepared samples were examined at ambient temperatures under a fluorescence microscope, Eclipse Ti-U (Nikon Instruments, Japan). The resulting cell images were then analyzed with NIS-Elements advanced research software Ver. 4.20 (Nikon Instruments) or Image J (NIH, USA).

### Preparation of Patterned Stamps

Closely spaced arrays of 10 µm x 2 µm x 10 µm (wide x height x spacing) were fabricated using a mask containing the defined dimensions and photo-lithography were used.

In certain embodiments, geometrical patterned shape-changing hydrogel arrays were fabricated using soft lithography as described in Akintewe et al. (2015) and DuPont et al. (2010). The resulting arrays of rectangular beams were covalently bound to a glass coverslip (22 mm x 22 mm) and possessed a spacing of 50 µm.In preparation for cell culture, the hydrogel arrays were coated with 50 µL of 0.1 mg/mL PLL for 5 minutes followed by rinsing in DPBS and then ambient drying in the biosafety hood for 2 hours prior to cell seeding.

### Cell culture

C2C12 and NIH3T3 cells were cultured in DMEM supplemented with 1% antibiotics (final concentration of 10,000 units/mL penicillin and 10,000 units/mL streptomycin) and 15% or 10% NCS growth medium, respectively, at 37°C in a humidified atmosphere of 5% CO₂. To prepare tissue precursors, trypsinized cells were seeded onto the hydrogel arrays at a density of 500 -750 cells/mm2 and cultured at 37 °C until confluence (24 to 48 hours).

### Statistical Analysis

Pair-wise comparisons for cell viability, alignment, and orientation data were performed using the two-tailed Student's t-test with n ≥ 3, wherep < 0.05 were considered statistically significant.

### EXAMPLE 1 - Compressive Pressure Applied During Printing Affects Cell Viability

Viability of the printed tissue precursors was found to be predominantly correlated with the amounts of compressive pressure applied during printing. Cell survival was observed after 24 hours of printed tissues on polylysine coated target surfaces. To examine the adequate amounts of pressure necessary to print tissue modules, cells seeded on the shape-changing structures were subjected to pressures ranging from 0.71 to 14.5 psi and viability of the cells after transfer was analyzed. Calibration weights of 1-g, 2-g, 5-g, 10-g, and 20-g were used to vary the amounts of pressure. A LIVE/DEAD viability assay determined that the lowest amount of pressure applied resulted in the most viable cells (-83% ± 3) of the (Figure 3). The number of dead cells decreased as the amount of pressure applied was systematically reduced. A plot of the measured viable cells shows a power series regression model with an R-squared value of 95.9%.

### EXAMPLE 2 - Effect of Micro-contact Printing on Cell Adhesion Molecules Adsorbed Target Surfaces

Printing on target surfaces adsorbed with collagen type 1, fibronectin, and polylysine did not show any statistical difference in cell viability. Viability of printed tissue precursors was reduced by 11, 22, and 7% for collagen, fibronectin, and polylysine, respectively, in comparison to the control samples. The negative control consisted of cells printed on plain glass surface, and no cell transfer was observed to such surfaces while a positive control that included cells cultured on plain glass showed the highest number of viable cells. A single factor ANOVA test indicated no significant difference between the means (p > 0.05). For subsequent printing, polylysine was selected as the CAM of choice for all target surfaces due to the ease of use and stability at ambient temperature.

### EXAMPLE 3 - Printed Tissue Precursors Reveal Preserved Focal Adhesion Components

To assess cellular morphology and metabolic activity, printed cells were compared to cells cultured on a plain glass surface. An hour after printing, the actin stress fiber staining showed an elongated morphology compared to the rounded morphology seen in cells seeded on glass even after 2 hours. To examine the cell-matrix adhesions, the cell nucleus was stained and the alpha 5 integrin observed an hour and 2 hours after printing. The results reveal emergence of physical integrin clustering at an hour after printing and enhanced clustering after 2 hours of printing indicating rapid reformation and recapitulation of focal adhesion components, which is attributed to the preserved cell morphology compared to the control samples (Figure 5).

### EXAMPLE 4 - Shape-changing Surface Provides Contact Guidance Cues for Cellular Organization

Analysis of cell orientation distribution revealed that cells elongated along the hydrogel pattern longitudinal direction well at 24 hours (Figure 6C-6D). Spatial organization of printed cells showed a preferred orientation between 0° and 30° axis, compared to the random distribution observed in cells cultured on non-patterned pNIPAAm hydrogel after 24 hours (Figure 6A-6B). Cell alignment calculations were made from the average cell orientation distribution values and the results indicate 90.8 % ± 0.11 of cells were aligned within ± 20° of the hydrogel array pattern direction.

Cellular organization was found to be dependent on the patterning of the hydrogel. Significant difference was observed between alignment of cells on non-patterned and patterned surfaces. Together these results indicate that the shape-changing hydrogel arrays provide contact guidance cues for spatial organization of cells depending on the width dimensions of the arrays.

### EXAMPLE 5 - Micro-contact Printing on C2C12 cells

The micro-contact printing technique described here can be used independent of cell types. C2C12 cells printed with shape-changing hydrogel stamps were observed to occur under the same conditions as the fibroblasts. Myoblast tissue modules were incubated for 15 minutes at 37°C and the tissue was completely transferrable under 1g weight to a target surface coated with polylysine within 5 minutes at 4°C (Figure 7). However, the skeletal myoblasts were observed to be more contractile, so precautions were taken to avoid premature release. Viability of the myoblasts was determined as 89 %.

### EXAMPLE 6 - Fabricating tissue precursors from shape-changing surfaces using micro-contact printing

The invention provides culturing cells on surface-confined poly-isopropylacrylamide hydrogel structures to produce functional tissue modules. In one example, 3T3 fibroblasts were used to demonstrate the feasibility of printing due to their immortalized properties.

Notably other forms of printing tissues from stimuli responsive surfaces have developed functional transplantable tissue for repairs of such disparate conditions as corneal dysfunction, periodontal ligament, and cardiac failure. However, these methods are limited to layer-by-layer assembly of tissues, which requires the use of longer incubation times up to 90 minutes at cold temperatures due to the hydration limitations of the nanoscale grafted brushes of the thermoresponsive surfaces used. Other printing approaches that include inkjet printing and mechanical extruders bioprinting technologies seem promising for fabrication of solid organ structures, owing to their high culture throughput, improved cell seeding densities, and controlled spatial resolution. Boland and others used a commercial, thermalbased inkjet printer in printing Chinese Hamster Ovary (CHO) cells into pre-defined patterns and were able to show improved cell viability, though patterning of the cells was not well maintained. In these studies, requirements of cell suspension intermediates; loss of cell viability; need for low viscosity solutions to prevent clogging during droplet generation; maintaining cell morphology and organization of cell patterning; and the relatively high cost of printers, still remains a challenge. An alternative approach is the use of a nozzle-free acoustic ejector to overcome clogging. Hydrogel droplet based systems or cell-suspension intermediates were found to address some of these challenges. Demirci and others developed a cell-laden hydrogel droplet deposition system for printing bladder smooth muscle cells and showed microscale spatial resolution while maintaining long-term cell viability.

The invention demonstrates printing cells from a simple and direct contact printing method. Similar to conventional micro-contact printing process whereby the stamp, typically a PDMS template, deforms while transferring the material of interest to its target, herein the stamp is a shape-changing structure that laterally expands around its transition temperature during cell printing. This expanding surface allows the stamp to be lifted off the target gradually, without smearing or distorting the patterned tissues. Cells grown on such surfaces were recently reported to detach as intact tissue stripes by an applied strain of at least 25%, as a result of lower adhesion strength between the ECM and shape-changing surface compared to the stronger bond between cell and its ECM. Sagging or roof collapse commonly reported in µCP is not experienced in the invention.

Fabrication of functional tissues via a printing mechanism requires processes that are not detrimental to the health of the cells, but rather that promote growth and preserve functionality *in vivo.* Pressure < 1 psi was found to yield approximately 83% in cell viability (Figure 3). Pressures lower than 0.71 psi impeded cell transfer due to insufficient conformal contact to the target surface.

The effects of protein or CAM adsorbed to the target surface using a compressive pressure of 0.71 psi are provided. All tissues were printed within 5 minutes. Collagen type 1 and human plasma fibronectin were selected based on their relative abundance physiologically, while polylysine, a chemically synthesized positively charged amino-acid, was selected to promote cell binding to culture surfaces by electrostatic interactions with the negative charged ions of the cells. No statistically significant difference in cell viability was observed between the CAMs tested, though the polylysine target surface showed higher amounts (Figure 4). Hence, all studies utilized polylysine for its ease of use and stability.

Cellular organization is crucial for controlling microarchitecture and biological functions of tissues. Therefore, the alignment and orientations of printed cells, as well as the effects of pattern width of the hydrogel, were evaluated. Cells proliferated randomly on non-patterned hydrogel. Meanwhile, preservation of spatial organization of cells was apparent after printing from the hydrogel arrays, indicating that the arrays provide contact guidance cues for cells to orient. The percentage of cells that oriented along the longitudinal axis of the shape-changing array differs depending on the width of the arrays, a result consistent with other findings. A 50 µm pattern width showed significantly increased alignment with 3T3 fibroblasts and normal human dermal fibroblasts as compared to wider patterns. Patterning dimensions for cell functionality is important and necessary for promoting cell-to-cell communication in cardiomyocytes, as reported by McDevitt. A specified width dimension of < 30 µm was observed to induce synchronous beatings in cardiomyocytes, an electrical coupling property necessary for fabrication of functional myocardial constructs.

Importantly, examination of the focal adhesion complexes of the printed tissue precursors was noticeable shortly after printing. These results suggest that the µCP printing technique does not hinder the normal regulation of cell binding and signaling activities. The actin filaments and alpha 5 integrin markers were observed to be significantly oriented in the cells compared to the control samples, suggesting that the cytoskeleton of the cell is also regulated by the hydrogel array patterning (Figure 5). This preservation of the cell-matrix adhesion domains allows for assembly of subsequent tissue stripes, thus enabling construction of 3D tissues.

C2C12 myoblast cells demonstrated similar detachment and printing times with the exceptions of the high contractility nature of the cell type. The cell stripes were observed to readily detach while pulling along neighboring cells at higher tension than with fibroblasts. This cell type can be used in sequential stacking of 3D cell stripe layers.

µCP printing of cells directly unto a surface without the need of an intermediate has been demonstrated. Functional tissue precursors can be printed within 5 minutes with stamps constructed from a shape-changing hydrogel of poly-*N*-isopropylacrylamide. The stamps provide contact guidance cues for tissue spatial organization as observed by the cellular organization along the major axis of the hydrogel array. Formation of focal adhesion complexes is prominently observed within a short time after printing. Tissue printing developed by a combination of shape-changing surfaces and µCP technique provides an alternative approach to other printing techniques, allowing for faster production and preserved organization. In addition, this process can be optimized to allow printing of unique shapes of tissues to aid vascularization upon tissue implantation. Fabrication of various geometries of the hydrogel arrays can provide contact guidance cues for assorted tissue formation.

Micro-contact printing of organized tissue modules from patterned arrays of shape changing hydrogel structures was demonstrated. Tissues are printed under low compressive pressures onto preferred target substrate without distorting the cell morphology or organization, while still preserving cellular functions. This method is easy to use and adaptable since no elaborate equipment is required, and therefore allows for translation to other forms of stimuli responsive systems.

Optimization and automation of the printing system to enable higher printing resolution and culture throughput while exploring printing of versatile patterns for development of tissues that have complex organized microarchitecture *in vivo* are also provided. This direct method of printing cells without the need of cell suspension intermediates provides a powerful tool for investigation of tissue morphogenesis *in vitro.*

### EXAMPLE 7 - Micro-contact Printing of Cells

Figure 8 shows the schematic diagram of the micro-contact printing technique. Pre-stained cells with density > 500 cells/mm² is seeded on polylysine coated patterned arrays of the shape-changing hydrogel. After 24 to 48 hours, the stamp containing confluent cells is inverted and placed in contact with a target surface to facilitate cell adhesion. Then a 1 or 2 gram calibration weight is taped to the back of the seeded stamp and placed on the target surface (monolayer of cells or pre-coated TCPS) in medium. The sample is then incubated for 15 minutes at 37°C and immediately transferred to 4°C chamber for 5 minutes. Then the weight is gradually lifted off the target surface leaving behind a cell stripe, while no cells are left on the stamp. The temperature reduction causes swelling of the hydrogel arrays inducing detachment of the tissue modules from the stamp to the target surface.

### EXAMPLE 8 - Assembly of Multilayered Patterned Cells

Two sets of double layer sheets were assembled on an unstained monolayer of confluent cells. Patterned stripes of myoblasts were directly printed on the two dishes of monolayers of fibroblasts. Collagen solution was cured on one of the monolayers. 100 µg/mL of bovine collagen type I with a pH of 7.3 was prepared by following commercially recommended protocol. 2 mL of the collagen solution was gradually added to a confluent cell culture dish after the medium was removed. The solution was then allowed to cure for 90 minutes at 37°C.

Triple-layer assemblies of tissue precursors were constructed by stacking patterned myoblasts in between patterned fibroblasts and a monolayer of confluent fibroblast. The patterned fibroblasts and myoblasts were pre-stained with cell tracker orange and green, respectively, and stacking was oriented 90° from each other.

### EXAMPLE 9 - Formation of Fibroblasts and Myoblasts Tissue Modules

Shape-changing hydrogel from pNIPAAm arrays is used to fabricate tissue modules of multiple cell types by employing the micro-contact printing technique. Cells printed from arrays of the shape-changing structures maintained their cell orientations either when printed on protein coated surfaces or a monolayer of cells (Figures 9-10). No observable differences were seen in the printing mechanism of the two cell types. Both cells released from the hydrogel in 5 minutes at 4°C under a compressive pressure of 1 g calibration weight. However, myoblast cells seem to elongate more along the longitudinal axis of the arrays as compared to fibroblasts.

### EXAMPLE 10 - Multilayered Assembly of Tissue Modules

Multilayered structures were achieved by stacking tissue modules onto a preexisting layer or layers of cells. Figure 11 shows the assembly of two layers of cells. A fibroblast cell stripe transferred onto a monolayer of fibroblast cells was first observed to print on the monolayer, as the cells in that layer seem to pave a pathway for the cell stripe (Figure 11A-11B). This occurrence was due to the lateral volume expansion of the hydrogel and the amount of compressive pressure on the arrays. Swelling of the hydrogel which is induced by the reduction in temperature at 4°C can expand the beam by a factor of 0.5 of its original size. The expansion in the hydrogel array is the observed print on the monolayer of cells at the top and bottom sides of the tissue array. Reduction in the compressive pressure to 1 g-calibration weight improved the transfer of the cell stripe (Figure 11C-11D). Printing a myoblast cell stripe on a monolayer of fibroblasts did not influence the printing resolution or outcome compared to the fibroblast cell stripe onto fibroblasts monolayer (Figure 11D-11E). Printing of intact tissue modules is attained on a 3-layered structure (Figure 12). The myoblast cell stripe is printed between a layer of fibroblast cell stripe and its monolayer. The 3-layer tissue module structure is located where the three boxes overlap.

### EXAMPLE 11 - Formation of Diverse Tissue Modules

Arrays of the shape-changing hydrogel allow for printing of tissue modules with different geometries and/or spacing. Fibroblasts cultured on stamps fabricated with closely spaced arrays of the microbeams maintained cell orientation along the long axis of the beams (Figure 13). Tissue module released from a 75 µm beam array resulted in a buckled structure when printed on protein adsorbed TCPS (Figure 14). Cells seeded on arrays with aspect ratios > 1.0 are expected to buckle due to conformance to the final structure of the hydrogel array in the swollen state. These results indicate that patterning of the hydrogel may be used to direct cellular organization of diverse forms.

### EXAMPLE 12 - Micro-contact printing for printing patterned arrays with shape-changing hydrogel

Fibroblast and myoblast cells were cultured independently on the microbeams of the shape-changing hydrogel while micro-contact printing technique was used to transfer the organized tissue modules into parallel and perpendicular orientations. The possibility of forming diverse tissues was also introduced. This example of the invention provides means for building and controlling complex tissue structures.

Cellular behavior is influenced by response to surface geometry and co-culture with other cell types. Cell reorganization and formation of vascular networks were observed when stripe-patterned endothelial cells were sandwiched between parallel oriented sheets of normal human dermal fibroblasts. However, a perpendicular orientation of the NHDF sheets did not reorganize the cells.

Tissue modules transferred to protein adsorbed TCPS maintained their cellular organization (Figures 9-10). The morphology of the cells was the same before and after printing. Myoblast cells were more elongated, depicting its normal anisotropic orientation *in vivo* while the fibroblast cells align along the longitudinal axis of the hydrogel array, indicating that the patterning of the hydrogel provides contact guidance cues for cell orientation. This organized cell orientation was, however, more unobtrusive in the multilayered construct (Figure 11-12). The 3-layered construct showed a change in cell morphology of the two cell stripes printed on the cell monolayer, though the basal cells maintained their cell organization. Cell reorganization in a multilayered system is commonly observed with multicellular systems, indicating that the geometry of the base surface can be used to dictate cellular behavior.

In Figure 11D, the staining color for both cell layers is uniform due to the infiltration of dye solution from the top to the bottom cell monolayer. To address this, concentration of the staining solution was significantly lowered. Then a solution of collagen was introduced between the layers to prohibit the infiltration. This change only significantly distorted the organization of the top cells. Finally, pre-staining the cells prior to culturing significantly improved the individual visualization of the cell layers. Most importantly, tissues printed from this shape-changing platform with micro-contact printing technique retained their biological activity as demonstrated in section 4.4.

The feasibility of printing tissues with different geometry was revealed. Cells printed from closely spaced arrays of 10 µm (Figure 13) maintained their cell organization while cells detached from a 75 µm wide microbeam (Figure 14) showed a well-defined buckled tissue structure. These results indicate that different patterning geometries of the shape-changing hydrogel can direct tissue structures into diverse multipurpose configurations. To control cell organization in the 3D multilayered tissue construct, cell reorientation, duration, influence of cell types, morphology, and cell density can be modulated. Confocal imaging can be used for evaluating the cross-section of the multilayered tissue construct. Also, different geometries of the shape-changing hydrogel can be fabricated to induce printing of tissues with diverse geometries for tissue engineering.

A micro-contact printing technique is employed in assembly of tissues in an additive manner to create multicomponent spatially controlled three-dimensional functional tissues. The invention provides methods of fabricating multilayered tissues with the flexibility of forming diverse tissue structures. The combination of shape-changing hydrogel and microcontact printing techniques allows a well-organized and rapid printing of tissue modules that can aid formation of native tissues with complex microarchitecture for regenerative medicine.

### EXAMPLE 13 - Assembly of 3D tissues

An example of the invention provides assembly of organized 3D tissues that closely represents native tissue, for example, cardiac tissues that closely represent the native myocardium. The 3D tissues of the invention provide vascular integration, diffusion, blood perfusion, and new capillary formation *in vivo,* thus revolutionizing regenerative therapies.

### EXAMPLE 14 - Automated Micro-contact Printing Mechanism

The invention provides a procedure suitable for printing cells; though the global organization and cell-to-cell junctions are sometimes not fully retained when transferred to the target surface, depending on the physical stability of the operator. The ability to control multi-cellular local and global organization is significant for producing 3D tissue constructs. To this end, the invention provides a robust method for reliable printing of functional cardiac tissues independent of the operator.

Automation of the micro-contact printing of cells cultured on shape-changing stamps enhances productivity and reduces errors caused by shearing of the cells during placing or removing stamp from the target surface. An enclosed motorized stage with a heating and cooling chambers provides for the contact printing of cells. LabView software can be used to record the printing parameters.

A micro-contact printing mechanism can be governed by conformal contact between a stamp and its target whereby cells efficiently transfer to the target with preserved cell viability and organization. The printing parameters include the contact time, release temperature, applied pressure, and the surface chemistry of the target. These parameters play a significant role in maintaining the intact cell stripe during and after transfer. The measurable outcome is the percentage of remaining attached cells on the stamp after transfer. An optimum printed tissue is a fully viable tissue module with the shortest amount of contact time independent of the aspect ratio or geometry of the patterned hydrogel.

To confirm the formation of intercalated discs, *i.e.* the adhering structure responsible for synchronized contraction of the myocardium, the cardiac tissues can be immunostained for desmosomes, cadherins, connexins, and the cell-to-cell junctions. Transmission electron microscopy (TEM) can be used to examine the intercellular junctions that indicate tissue formation. Video via optical microscopy can be used to monitor changes in cell morphology, while the NIH Image J software can be used to analyze cell alignment and orientation. Cytotoxicity assay containing Calcein AM and ethidium homodimer can be used to evaluate cell viability.

### EXAMPLE 15 - Optimized Design Parameters of the Stamp

The width and spacing of laminin patterns were found to affect the alignment and orientation of contractile myofibrils, and the formation of cell-to-cell junctions. Adherent cardiomyocytes (CMs) on narrowly spaced patterns of 10µm and widths < 20µm were found to maintain cell alignment and also yield synchronized contractions on laminin coated surfaces. Closely spaced arrays (10 µm) of the shape-changing arrays yield aligned tissue modules along the longitudinal axis of the target surface (Figure 13). To determine myofibril alignment and synchronous beatings of CMs, the optimal adhesive domain geometry for cellular organization can be evaluated. In addition, cell morphology, organization and formation of myofibrils are influenced by the rigidity of the substrate; the stiffness of the substrate can drive the tension forces between cells. Thus, the hydrogel properties can be optimized by varying the densities of crosslinks. Lower crosslink density gels are softer and have more degrees of freedom to swell, leading to more rapid cell sheet recovery. However, a high crosslink density gel can induce the stiffness required for the normal mechanical workload of the myocardium. Since organization of CM is geometric and substrate stiffness dependent, the effects of PNIPAAm array spacing and crosslink density can readily be optimized.

The effect of array spacing and modulus on the organization, rate of release, and pulsation of cardiomyocytes can be assessed. Photolithography can be used to fabricate master molds with new sets of array spacing. The aspect ratio of the arrays can remain constant while the spacing and crosslink densities can vary from 5-20 µm and 1-4% respectively. Atomic force microscopy can be used to evaluate the mechanical force of the arrays. Primary cardiac myocytes can be cultured on these arrays and their local and global organization can be examined over a prolonged culture using fluorescence microscopy and the NIH image J software for analysis. The ease of rapid tissue transfer can be assessed by studying the release kinetics induced by a 10°C shift below the culture temperature. The release kinetics can be monitored on a video via confocal microscopy. Electrocardiogram (ECG) can be used to evaluate the synchronous beating of the engineered CMs. As such, optimal PNIPAAm patterns direct myocytes organization and regulate their contractions.

Cytoskeletal organization and expression can be evaluated by immunofluorescence staining with actin phalloidin, cell nuclei stain-Hoechst 33342, and antibodies to myosin. To confirm the formation of intercalated discs, the optimal cardiac tissue can be immunostained for desmosomes, cadherins, and connexins, the cell-to-cell junctions responsible for synchronized contraction of the myocardium. TEM should be used to characterize the myofibril structure and the cell junction morphologies.

### EXAMPLE 16 - Constructing functional 3D tissues

Prevascularization of thick grafts promote *in vivo* perfusion, neovascularization, and angiogenesis in myocardial ischemia and infarction of rats. Diffusion of nutrients is limited to a graft thickness of 80 µm.Multiple surgeries were used to overcome this drawback. However, repeated surgeries (-10 times) within 48 hours are not feasible for humans. As a result, an alternative strategy to enhance diffusion is necessary. Human umbilical vein endothelial cells (HUVEC) can differentiate into beating CMs when co-cultured with neonatal rat CMs with high efficiency (11 ± 0.5%). Thus, HUVEC can be stacked between CMs to promote neovascularization (Figure 15).

3D cardiac tissues can be assembled by sequential printing of HUVEC between layers of CMs via the contact printing procedure. The CMs should be cultured on the optimal narrowly spaced arrays, while the HUVEC can be on widely spaced arrays to support vascular lumen formation. To promote diffusion, different stacking orientations (parallel and perpendicular) and ratios (CM :HUVEC :CM = 3:1:3, 3:2:3, 3:3:3, 4:2:4) can be used. Confocal microscopy can be used to analyze the corresponding thickness and examine the formation of cell-to-cell junctions between layers of adjacent cells. CellTracker dyes can be used to differentiate between layers. The engineered 3D construct can be maintained in static cultures for up to 2 weeks with daily monitoring and examination of physical properties.

Immunohistology assays can be used to examine the expressed cardiac markers, cell viability, organization, morphology, and vasculogenesis of the tissue construct. To stimulate cardiac neovascularization perfusion culture with growth factors supplements can be performed. This culture can be monitored in a flow chamber with the suspended tissue aligned parallel to the flow. Pulsation is crucial for cardiac function, as the heart beats -100,000 times daily. Native myocardium undergoes repetitive forces during contraction, hence the tissue construct can be designed to withstand compressive forces up to 3 mN/mm². Atomic force microscopy can be used to analyze the engineered tissue compressive forces and an electrocardiogram can be employed to assess the synchronous contraction of the engineered cardiac muscles.

### EXAMPLE 17 - Transfer printing of tissue precursors via geometrically patterned shape-changing hydrogels

In vitro fabrication of tissues is sought for developing platforms to screen candidate drugs and study tissue morphogenesis, and ultimately to regenerate damaged organs. A modified microcontact printing technique is provided where patterned shape-shifting poly(N-isopropylacrylamide) hydrogels was used to print organized 3T3 fibroblast tissue precursors to target surfaces coated with either fibronectin, collagen type 1 or poly-L-lysine. The patterned hydrogels directed cell-organization on the stamp, and a subsequent shape-shift of the hydrogel triggered instantaneous release of the tissue precursor from the stamp. Post printing, the tissue precursors underwent rapid repolarization with the emergence of focal adhesion complexes on a time-scale much faster compared to cells seeded by standard sedimentation. Moreover, a minimum stamp pressure was necessary for transfer; however, at stamp pressures above approximately 1 psi, cell viability was strongly compromised. To demonstrate the ability to print other cell types using this transfer printing technique, skeletal myoblasts were also printed. These results suggest that tissue modules can be organized and transferred with intact pre-patterned morphologies from shape-changing hydrogel structures using a microcontact printing technique. This approach enables the bottom-up construction of high cell density scaffold-free tissues with programmed configuration down to the individual cell level.

A major stumbling block for *in vitro* tissue engineering is the inability to recapitulate cell dense constructs with the necessary diversity in cell types and organizational complexity to mimic native tissues. 3D printing offers the promise to overcome such challenges; however the two dominant tissue printing approaches, including inkjet and extrusion-based technologies, have inherent limitations. In particular, neither are well-suited to printing *a priori* physiologically organized cellular precursors. Both require the use of non-cellular intermediates to suspend cells or multiceullar aggregates prior to printing, which may include cell culture media or hydrogel precursors. Despite convenience, a severe limitation of thermal or piezoelectric inkjet printers is the heat or shear forces at the printhead nozzle, which impairs cell functionality and reduces the viability of printed cell populations. This can be overcome in extrusion-based bioprinting techniques; however, the non-cellular intermediates still lack the combined spatial and temporal resolution of *in vivo* physical and chemical cues essential for physiological coalescence and organization. A further complication of the use of intermediates is a reduction in printed cell density. Although both the inkjet and extrusion-based technologies have several potential benefits with regards to tissue engineering and regenerative medicine, layer-by-layer printing of organized pre-tissue constructs without the use of intermediates offers a path towards rapid formation of tissues with preserved cell metabolic activity and morphology and significantly greater cell density.

A tissue fabrication method whereby cell sheets released from smart culture dishes are assembled into laminar tissues with a manipulator stacking technique is known. This group has demonstrated the feasibility of constructing transplantable multilayer tissues that have functioned to promote vascularization and recover diminished vision. The cell sheet approach relies on electron beam polymerization of ultrathin poly-*N*-isopropylacrylamide (PNIPAAm) hydrogel coatings directly on polystyrene tissue culture dishes. Cellular alignment on the PNIPAAm coating may be achieved through secondary modification by fibronectin patterning. The cell sheet is released by a thermally-induced hydrophobicity change, which requires the use of long incubation times (up to 90 minutes). Cells that have been metabolically poisoned do not release, indicating that metabolic processes including cytoskeletal reorganization are required for the process to be successful. Cell sheet transfer from thermoresponsive wrinkling surfaces is also known.

An embodiment of the invention provides an alternative layer-by-layer method for the assembly and transfer of patterned cells via thermally mediated shape-shifting responsive substrates by employing a cell transfer analogue of the microcontact printing technique. There are two potential advantages to the shape-shifting stamp approach. First, no secondary chemical patterning is necessary for alignment, which is now determined by contact guidance of the stamp edges. Second, release is rapid (in the order of seconds) which limits loss of cell alignment or morphology changes during the contact printing process.

Microcontact printing (µCP) technology was developed over two decades ago for creating patterns of thin gold features on surfaces at micron lateral length scales. Since then, this technique has proven invaluable for functional patterning of surface chemistries and biomolecules, and has enabled investigations of cell shape control in cell survival, differentiation, and adhesion strength. µCP relies on the use of a deformable polymer stamp with a relief pattern and an ink that is transferred from the protruding stamp features to the target surface upon contact. Typically, the inks are chemicals that react with the target surface or proteins that adsorb. To our knowledge, transfer printing of patterned cells directly from stamps onto target substrates has yet to be illustrated.

An embodiment of the invention provides the use of patterned cell sheets and the process parameters that regulate viability and pattern transfer fidelity. Tissue modules adhered to arrays of shape-changing three-dimensional (3D) poly-*N*-isopropylacrylamide (pNIPAAm) hydrogel patterns are detached through a strain-mediated process and that the release occurs almost instantaneously. Combination of the shape changing hydrogel and µCP technique provides a reliable method for manipulating tissue precursors while maintaining their morphology and cell-cell contacts; a first step toward functional tissue assembly *in vitro.* Fibroblasts or skeletal myoblasts were cultured on fabricated 3D patterned pNIPAAm hydrogels and the effects of contact on transferred tissues, e.g., the effect of stamping pressure on cell viability and cell alignment, were investigated. The effects of cell adhesion promoters adsorbed on the target printing surface were also tested. Additionally, the assembly of cell-matrix adhesive contacts in the printed cells was evaluated. As such, a modular approach to bottom-up fabrication of cell-dense tissues composed of living microcontact printed building blocks is provided.

Microcontact printing from shape changing stamps with patterned tissue modules were used as the 'ink.' Printing tissue precursors from shape changing stamps is providing using intact patterned tissue modules released from shape changing surfaces. Placing the stamp so that the cell-covered relief pattern was in contact with a target surface and then reducing the temperature enabled transfer of the cell pattern onto the target surface (Figure 16A). Frequent failures, in the form of partial or incomplete transfer, during initial experiments motivated the investigation of the factors that govern strain-mediated tissue µCP.

Compressive pressure applied during printing affects cell viability. Complete transfer during µCP required conformal contact between the inked features of the stamp and the receiving target. External pressure was required to achieve conformal contact; however, excessive pressure compromised cell viability. To evaluate the effect of printing pressure, cells patterned on arrays of the shape-changing structures were subjected to pressures ranging from 0.71 to 14.5 psi for 5 minutes and the completeness of transfer and viability of the transferred cells were analyzed. The pressure was calculated as the total force distributed across the relief pattern features in the collapsed state. Viability of the printed tissue precursors predominantly correlated with the amounts of compressive pressure applied during printing onto PLL-coated surfaces (Figure 16). A LIVE/DEAD viability assay determined that the lowest pressure tested (0.71 psi) resulted in the preservation of the greatest cell viability; approximately 79 ± 7% remained viable (Figure 16). The number of dead cells increased as the amount of pressure applied was systematically increased. Below 0.71 psi, printing was incomplete. Maximum cell transfers also occurred at the lowest applied pressures up to 4 psi. Pressures greater than 4 psi resulted in significantly lower pattern transfer and fragments of cell bodies could be observed when the stamp was removed. Thus 0.71 psi was the optimal pressure for transfer and survival for this cell type and stamp relief pattern combination.

Effect of target matrix coating on printed cell viability was examined. A variety of natural and synthetic target matrices were investigated since poor transfer occurred onto clean, unmodified glass surfaces. Using a printing pressure of 0.71 psi, fibroblast tissue precursors were printed onto glass surfaces pre-coated with adsorbed collagen type 1, fibronectin or PLL and the viability of printed cells was quantified with a LIVE/DEAD assay (Figure 4). In comparison to the control samples (cells seeded on plain glass in serum for 24 h), viability of printed tissue precursors was reduced by 11, 22, and 7% for collagen, fibronectin and PLL, respectively. However, viability was only significantly reduced compared the control for the collagen and fibronectin surfaces (p < 0.01), and differences among treatments were not significant. This result confirms that a variety of extracellular matrices (ECM) can be used as target surfaces for µCP. In contrast, cell transfer onto clean glass surfaces without a matrix coating largely failed to transpire within 5 minutes. For subsequent printing, PLL was selected as the matrix of choice for all target surfaces due to the ease of use and stability of this coating.

Cellular organization and alignment from stamp contact guidance cues was maintained after µCP. Since the cells in the tissue precursors were transferred directly from a patterned surface, morphological characteristics would be maintained through the µCP process. Cells primarily aligned with the stamp's relief pattern prior to printing. An analysis of cell orientations after printing revealed that this alignment was preserved after µCP (Figure 6). In contrast to the random distribution observed in cells cultured on non-patterned pNIPAAm hydrogel coatings or seeded directly onto target surfaces, the long axis of cells on the target 1 hour after printing showed a strongly preferred orientation parallel to the now-removed stamp pattern. Based on the average cell orientation distribution values from multiple experiments, 90.8 ± 0.1% of the cells were aligned within ± 20° of the stamp pattern direction.

Preserved morphology in printed tissue precursors enhanced cell adhesion and cytoskeletal maintenance. Features of spread cells, including actin stress fibers and adhesion structures, were analyzed. Cells printed onto PLL-coated surfaces were compared to cells seeded by standard sedimentation procedures to assess cellular morphology and cell-matrix adhesion assembly (Figure 17). 10 minutes after printing tissue precursors, asymmetric spread morphologies and continuous multicellular stripes were maintained through the printing process as observed by the preservation of cytoplasmic actin structures. Nascent focal adhesions had already begun to appear in the form of integrin clusters. 1 hour after printing, focal adhesions and actin stress fibers were more pronounced, and by 2 hours after printing, focal adhesions were numerous and associated with the actin stress fibers which primarily were oriented along the longitudinal direction of the tissue precursors. In contrast, after 10 minutes to 1 hour, cells settled onto the surface from solution had rounded shapes and no organized actin filaments or focal adhesions. After 1-2 hours, seeded cells were just beginning to spread, assembled focal adhesions and displayed limited cytoskeletal organization.

### EXAMPLE 18 - Fabricating and transferring patterned tissue precursors from PNIPAAm shape-changing stamps using µCP

Viability of fabricating and transferring patterned tissue precursors from PNIPAAm shape-changing stamps using µCP is provided. A stamp, typically a PDMS template, transfers the ink material of interest to its target. Herein, the ink is a patterned layer of cells and matrix, and the stamp is an array of shape-changing hydrogel structures that laterally expand and strain the attached tissue precursors during transfer. 3T3 fibroblasts patterned on such stamps could be detached as intact strips when the hydrogels underwent a lateral strain of at least 25%, which cleaved the cell-ECM bonds but preserved cell-cell contacts. Thus, transfer was enabled by allowing the patterned cell ink to adhere to the target and then release from the stamp by initiating lateral strain in the stamp's hydrogel relief pattern. Cell sheets have been released and transferred from PNIPAAm grafted surfaces in another process; however, that process does not allow the discrete patterning of the cell layers which is a hallmark of µCP. Also, release from the stamp works by a notably different mechanism. Further, sagging or roof collapse commonly reported in µCP is not experienced in the method described herein.

Surface chemistries of the target substrate and the amounts of compressive pressure applied during transfer were found to affect printing in the methods described herein. The effect of the compressive pressure on the printing of fibroblast tissue precursors was studied. Notably, a minimum pressure was needed (0.71 psi) to provide reliably sufficient contact for transfer. At pressures lower than 0.71 psi, transfer was inconsistent and sometimes did not occur at all. Most likely, the tissue precursor does not make uniform contact with the receiving surface. In these cases, the tissue precursors either rolled into clumps or were only partially attached to the receiving surface after printing. In such cases, the transferred tissue precursor had a substantially different structure than what was observed on the stamp prior to transfer. At the minimum pressure to reliably provide transfer, cell viability was approximately 83% (Figure 16). At higher pressures (up to 4 psi), tissue precursors could still be completely transferred, however, cell viability significantly suffered.

Indeed, control studies showed that confluent layers of 3T3 cells experienced increased death due to compressive pressure for 0.88 psi and greater. This is significant on two fronts. First, the lateral strain that cells endure during the shape shift of the stamp is not responsible for cell rupture. Second, any sort of transfer printing process must minimize the applied compressive stresses on the cells. In the case of printing at 0.71 psi, while some cell death occurred, the shapes and cytoskeletal organization of cells was largely preserved. Within 10 minutes to 2 hours of printing, significant focal adhesion reformation was observed, signifying that the cells in printed tissue precursors undergo rapid repolarization. Interestingly, this repolarization is faster than non-printed cells that sedimented onto PLL-coated surfaces, where previous studies show that focal adhesion formation is not robust until after 4 hours. Consequently, the rapid reformation and recapitulation of focal adhesion complexes in printed tissue precursors most likely stems from the preservation of the cell morphology after printing. Indeed, minutes after printing the tissue precursors, the actin stress fibers showed an elongated cell morphology compared to the rounded morphology observed in cells seeded on PLL-coated surfaces (non-printed cells) even after 2 hours in culture (Figures 6 and 17). To examine the repolarization and cell-target adhesion evolution, alpha 5 integrins was stained immediately after printing (10 minutes), and after 1 and 2 hours. Integrin clustering emerged as rapidly as 10 minutes and enhanced clustering was observed 2 hours post printing (Figure 17).

To optimize the cell viability and to understand which surface is more energetically favorable for rapid cell binding during transfer, the effects of adhesive molecules adsorbed to the target surface were studied. Collagen type 1 and human plasma fibronectin were selected based on their relative abundance and adhesive function in native tissues. In addition, PLL, a synthetic positively charged amino acid chain, was selected because it enhances cell adhesion to culture surfaces by electrostatic interactions with the negative charged surface of cells or by altering serum protein adsorption. No statistically significant difference in cell viability was observed among the adhesion factors tested, though the PLL target surface was greatest (Figure 4).

µCP described herein was tested in another cell type. Myoblasts were chosen to study potential applications in skeletal muscle tissue engineering. Patterned muscle tissue precursors of these cells demonstrated detachment and printing transfer speed similar to fibroblasts under comparable conditions.

µCP of pre-patterned tissue modules from stamps consisting of patterned arrays of shape changing hydrogel structures is demonstrated. The relief pattern of the stamp specifies the global shape of the tissue modules, and may also provide contact guidance cues that locally align cells and control their shapes, as observed by the cellular organization along the major axis of the hydrogel array features. The efficiency and fidelity of transfer depends on the pressure applied during stamping; some pressure is required to ensure conformal contact, but too much pressure injures cells. With optimization, tissue modules were printed under low compressive pressures onto preferred target substrates within 5 minutes. Like previous applications of µCP, this method is easy to adopt and versatile so that variety of tissue precursors could be printed using stamps responsive to numerous stimuli.

Methods described herein provide the ability to pre-pattern cells and then print them while maintaining their shapes and intercellular organization. As a result, rapid repolarization and adhesion occur without major reorganization. Furthermore, focal adhesion complexes are prominently observed within a short time after printing which reinforces the specified intra- and intercellular organization. Thus, the methods described herein provide a technology for three-dimensional stacking of patterned cell layers to construct tissues *in vitro.*

### REFERENCES

[1] Xu T, Zhao W, Zhu JM, Albanna MZ, Yoo JJ, Atala A. Complex heterogeneous tissue constructs containing multiple cell types prepared by inkjet printing technology. Biomaterials. 2013;34:130-9.
[2] Xu T, Jin J, Gregory C, Hickman JJ, Boland T. Inkjet printing of viable mammalian cells. Biomaterials. 2005;26:93-9.
[3] Lorber B, Hsiao W-K, Hutchings IM, Martin KR. Adult rat retinal ganglion cells and glia can be printed by piezoelectric inkjet printing. Biofabrication. 2014;6:015001.
[4] Jakab K, Norotte C, Marga F, Murphy K, Vunjak-Novakovic G, Forgacs G. Tissue engineering by self-assembly and bio-printing of living cells. Biofabrication. 2010;2:022001.
[5] Jakab K, Norotte C, Damon B, Marga F, Neagu A, Besch-Williford CL, et al. Tissue engineering by self-assembly of cells printed into topologically defined structures. Tissue Eng Part A. 2008;14:413-21.
[6] Demirci U, Montesano G. Single cell epitaxy by acoustic picolitre droplets. Lab Chip. 2007;7:1139-45.
[7] Lee V, Singh G, Trasatti JP, Bjornsson C, Xu X, Tran TN, et al. Design and fabrication of human skin by three-dimensional bioprinting. Tissue engineering Part C, Methods. 2014;20:473-84.
[8] Kikuchi T, Shimizu T, Wada M, Yamato M, Okano T. Automatic fabrication of 3-dimensional tissues using cell sheet manipulator technique. Biomaterials. 2014;35:2428-35.
[9] Kushida A, Yamato M, Konno C, Kikuchi A, Sakurai Y, Okano T. Temperature-responsive culture dishes allow nonenzymatic harvest of differentiated Madin-Darby canine kidney (MDCK) cell sheets. J Biomed Mater Res. 2000;51:216-23.
[10] Shimizu T, Sekine H, Yang J, Isoi Y, Yamato M, Kikuchi A, et al. Poly surgery of cell sheet grafts overcomes diffusion limits to produce thick, vascularized myocardial tissues. Faseb J. 2006;20:708-10.
[11] Muraoka M, Shimizu T, Itoga K, Takahashi H, Okano T. Control of the formation of vascular networks in 3D tissue engineered constructs. Biomaterials. 2013;34:696-703.
[12] Sasagawa T, Shimizu T, Sekiya S, Haraguchi Y, Yamato M, Sawa Y, et al. Design of prevascularized three-dimensional cell-dense tissues using a cell sheet stacking manipulation technology. Biomaterials. 2010;31:1646-54.
[13] Nishida K, Yamato M, Hayashida Y, Watanabe K, Yamamoto K, Adachi E, et al. Corneal reconstruction with tissue-engineered cell sheets composed of autologous oral mucosal epithelium. New England Journal of Medicine. 2004;351:1187-96.
[14] Tang Z, Akiyama Y, Okano T. Temperature-Responsive Polymer Modified Surface for Cell Sheet Engineering. Polymers. 2012;4:1478-98.
[15] Kumar A, Whitesides GM. Features of gold having micrometer to centimeter dimensions can be formed through a combination of stamping with an elastomeric stamp and an alkanethiol "ink" followed by chemical etching. Applied Physics Letters. 1993;63:2002-4.
[16] Singhvi R, Kumar A, Lopez GP, Stephanopoulos GN, Wang DI, Whitesides GM, et al. Engineering cell shape and function. Science. 1994;264:696-8.
[17] Jang M, Nam Y. Aqueous micro-contact printing of cell-adhesive biomolecules for patterning neuronal cell cultures. BioChip J. 2012;6:107-13.
[18] Akintewe OO, DuPont SJ, Elineni KK, Cross MC, Toomey RG, Gallant ND. Shape-changing hydrogel surfaces trigger rapid release of patterned tissue modules. Acta Biomater. 2015;11:96-103.
[19] DuPont Jr SJ, Cates RS, Stroot PG, Toomey R. Swelling-induced instabilities in microscale, surface-confined poly(N-isopropylacryamide) hydrogels. Soft Matter. 2010;6:3876-82.
[20] Akintewe OO, DuPont SJ, Elineni KK, Cross MC, Toomey RG, Gallant ND. Shape-changing hydrogel surfaces trigger rapid release of patterned tissue modules. Acta biomaterialia. 2015;11:96-103.
[21] Alom Ruiz S, Chen CS. Microcontact printing: A tool to pattern. Soft Matter. 2007;3:168-77.
[22] Gallant ND, Michael KE, Garcia AJ. Cell adhesion strengthening: Contributions of adhesive area, integrin binding, and focal adhesion assembly. Molecular Biology of the Cell. 2005;16:4329-40.
[23] Aubin H, Nichol JW, Hutson CB, Bae H, Sieminski AL, Cropek DM, et al. Directed 3D cell alignment and elongation in microengineered hydrogels. Biomaterials. 2010;31:6941-51.
[24] Takahashi H, Nakayama M, Itoga K, Yamato M, Okano T. Micropatterned thermoresponsive polymer brush surfaces for fabricating cell sheets with well-controlled orientational structures. Biomacromolecules. 2011;12:1414-8.

## Claims

1. A method of producing a tissue module, the method comprising:
a) seeding a basal surface with a first group of cells and incubating the first group of cells on the basal surface for a sufficient period of time to allow the first group of cells to adhere to the basal surface and produce a first layer;
b) seeding a thermo-responsive hydrogel stamp with a second group of cells and incubating the second group of cells on the stamp for a sufficient period of time to allow the cells to adhere to the stamp and produce a second layer;
c) contacting the stamp to the basal surface;
d) releasing the cells from the stamp by applying a change in temperature;
e) incubating the stamp and the basal surface for a sufficient period of time for the second layer of cells from the stamp to adhere to the first layer of cells on the basal surface; and
f) separating the stamp and the basal surface to release the tissue module,
wherein the method further comprises repeating b) to f) with a plurality of layers of cells and stacking the plurality of layers of cells onto layers of cells previously produced.

2. The method according to claim 1, wherein the arrangement of cells in the plurality of layers of cells mimics the arrangement of cells in a tissue or an organ.

3. The method according to claim 1, wherein the different layers of cells in the plurality of layers of cells comprise different types of cells, which mimic the types cells in a tissue or organ.

4. The method according to claim 1, wherein the basal surface comprises poly(dimethyl siloxane) (PDMS), optionally coated with an extracellular matrix component.

## Patentansprüche

1. Verfahren zum Herstellen eines Gewebemoduls, wobei das Verfahren Folgendes umfasst:
a) Aussäen einer basalen Oberfläche mit einer ersten Gruppe von Zellen und Inkubieren der ersten Gruppe von Zellen auf der basalen Oberfläche für einen ausreichenden Zeitraum, um es der ersten Gruppe von Zellen zu ermöglichen, an der basalen Oberfläche zu haften und eine erste Schicht herzustellen;
b) Aussäen eines thermoresponsiven Hydrogel-Stempels mit einer zweiten Gruppe von Zellen und Inkubieren der zweiten Gruppe von Zellen auf dem Stempel für einen ausreichenden Zeitraum, um es den Zellen zu ermöglichen, an dem Stempel zu haften und eine zweite Schicht herzustellen;
c) Berühren des Stempels mit der basalen Oberfläche;
d) Lösen der Zellen von dem Stempel durch Anlegen einer Temperaturänderung;
e) Inkubieren des Stempels und der basalen Oberfläche für einen ausreichenden Zeitraum, damit die zweite Zellschicht von dem Stempel an der ersten Zellschicht auf der basalen Oberfläche haftet; und
f) Trennen des Stempels und der basalen Oberfläche, um das Gewebemodul zu lösen,
wobei das Verfahren ferner ein Wiederholen von b) bis f) mit mehreren Zellschichten und ein Stapeln der mehreren Zellschichten auf zuvor hergestellte Zellschichten umfasst.

2. Verfahren nach Anspruch 1, wobei die Anordnung von Zellen in den mehreren Zellschichten die Anordnung von Zellen in einem Gewebe oder Organ nachahmt.

3. Verfahren nach Anspruch 1, wobei die unterschiedlichen Zellschichten in den mehreren Zellschichten unterschiedliche Zellarten umfassen, die die Zellarten in einem Gewebe oder Organ nachahmen.

4. Verfahren nach Anspruch 1, wobei die basale Oberfläche Poly(dimethylsiloxan) (PDMS) umfasst, optional mit einer extrazellulären Matrixkomponente beschichtet.

## Revendications

1. Procédé de production d'un module tissulaire, le procédé comprenant :
a) l'ensemencement d'une surface basale avec un premier groupe de cellules et l'incubation du premier groupe de cellules sur la surface basale pendant une période de temps suffisante pour permettre au premier groupe de cellules d'adhérer à la surface basale et produire une première couche ;
b) l'ensemencement d'un tampon hydrogel thermosensible avec un second groupe de cellules et l'incubation du second groupe de cellules sur le tampon pendant une période de temps suffisante pour permettre aux cellules d'adhérer au tampon et produire une seconde couche ;
c) la mise en contact du tampon avec la surface basale ;
d) la libération des cellules du tampon en appliquant un changement de température ;
e) l'incubation du tampon et de la surface basale pendant une période de temps suffisante pour que la seconde couche de cellules du tampon adhère à la première couche de cellules sur la surface basale ; et
f) la séparation du tampon et de la surface basale pour libérer le module tissulaire,
le procédé comprenant en outre la répétition des étapes b) à f) avec une pluralité de couches de cellules et l'empilement de la pluralité de couches de cellules sur des couches de cellules précédemment produites.

2. Procédé selon la revendication 1, dans lequel la disposition des cellules dans la pluralité de couches de cellules imite la disposition des cellules dans un tissu ou un organe.

3. Procédé selon la revendication 1, dans lequel les différentes couches de cellules dans la pluralité de couches de cellules comprennent différents types de cellules, qui imitent les types de cellules dans un tissu ou un organe.

4. Procédé selon la revendication 1, dans lequel la surface basale comprend du poly(diméthylsiloxane) (PDMS), éventuellement revêtu d'un composant de matrice extracellulaire.
